Europäisches Patentamt

(19) European Patent Office

Office européen des brevets

(11) Publication number: **0 012 617**
**B1**

(12) **EUROPEAN PATENT SPECIFICATION**

(45) Date of publication of patent specification: **29.12.82**

(51) Int. Cl.³: **G 01 N 33/00**

(21) Application number: **79302904.2**

(22) Date of filing: **14.12.79**

(54) Method for calibrating analytical moisture measuring equipment.

(30) Priority: **15.12.78 US 970048**

(43) Date of publication of application:
**25.06.80 Bulletin 80/13**

(45) Publication of the grant of the patent:
**29.12.82 Bulletin 82/52**

(84) Designated Contracting States:
**BE FR GB IT SE**

(56) References cited:
**US - A - 3 247 702**
**US - A - 3 804 595**
**US - A - 3 894 419**
**US - A - 4 056 966**

(73) Proprietor: **WESTINGHOUSE ELECTRIC CORPORATION**
**Westinghouse Building Gateway Center**
**Pittsburgh Pennsylvania 15222 (US)**

(72) Inventor: **Chinault, John Curtis, Jr.**
**112 Lamar Lane**
**West Columbia, South Carolina (US)**
Inventor: **Pregnall, Richard Alexander**
**140 Garden Springs Road**
**Columbia, South Carolina (US)**

(74) Representative: **van Berlyn, Ronald Gilbert**
**23, Centre Heights**
**London, NW3 6JG (GB)**

Courier Press, Leamington Spa, England.

Method for accurately determining the moisture content of
nuclear fuel pellets

The invention described herein relates to a method of measuring small amounts of water in nuclear fuel pellets.

A conventional nuclear reactor fuel rod of the type used in commercial nuclear reactors comprises a long narrow tube of zircaloy having multiple fuel pellets sealed therein. When the fuel rod is irradiated during operation of the reactor, hydrogen and other gases are released as a by-product of the fission process, but these gases are contained in the sealed rod and do not escape to the surrounding superheated water which ultimately is used to generate steam for power generating purposes. It has been found that excessive hydrogen in the fuel pellets appearing mostly as moisture, causes hydriding of the zircaloy fuel rod when the hydrogen is released from the fuel pellet during operation of the reactor. The resulting corrosive effects of the hydriding may cause a breach in the fuel rod material and result in radioactive particles leaking into the water circulating through the reactor.

To overcome this disadvantage, the upper limit set on permissible moisture content in a pellet, or more appropriately, the total moisture equivalent of hydrogen in all forms, was set at 10.5 ppm or less for an average of a region of fuel. In the past, the LECO Corporation, St. Joseph Michigan, commercial hydrogen determinator, Model RH1E, was used for measuring hydrogen alone while a CEC (Consolidated Electrodynamics Corporation) moisture determinator was used for measuring moisture in the moisture content of fuel pellets. The LECO determinator operating temperature is sufficiently high to dissociate water and is therefore capable of measuring the hydrogen content in moisture. However, the determinator may not always be operating accurately. The degree of precision needed in measuring finite amounts of hydrogen, both alone and from the water molecule, dictates that a single, high temperature moisture standard be utilized to measure total hydrogen, including moisture.

It is therefore the principal object of the present invention to provide a standard for the measurement of small amounts of water which can be used in connection with measuring the moisture content of nuclear fuel pellets.

With this object in view the present invention resides in a method of determining the hydrogen content of nuclear fuel pellets wherein a nuclear fuel pellet whose hydrogen content is to be measured is placed into a hydrogen measuring apparatus in which said pellet is exposed to a temperature sufficient to assure the extraction of moisture from said pellet, and the content of hydrogen in the moisture extracted from said pellet is measured and a readout thereof is provided on said apparatus, characterized in that, before the water content of said fuel pellet is measured, kaolinite having a water of crystallization of a known amount is surface-dried and sealed and is then heated in said apparatus to the temperature at which the water of crystallization is driven from the kaolinite, the quantity of hydrogen in the moisture driven from the kaolinite is measured and compared with the quantity of hydrogen known to be in the water of crystallization of said kaolinite and the apparatus is then precisely adjusted so as to provide an accurate reading for the following measuring of the water content of the fuel pellet.

It is known from U.S. Patent 4,056,966 to provide a standard for hydrogen content measurement on the basis of the known water bound within a chemical compound. Fuel pellets however are at very high temperatures during operation and it is at this temperature at which the moisture content must be measured.

The water of crystallization in the kaolinite which amounts to less than one milligram of water is known and, upon exposure to a high temperature in a confined area, the hydrogen in the moisture can be precisely measured for assuring accuracy in the measuring process by directly using the hydrogen content of the kaolinite as a comparison basis for the hydrogen content in nuclear fuel pellets.

The invention will become more readily apparent from the following description of a preferred embodiment thereof shown, by way of example only, in the accompanying drawing wherein:

The single figure is a graph showing the percent water in 18 samples of kaolinite and are compared statistically to the theoretical value of water known to be in kaolinite.

To accurately determine the hydrogen content in moisture, water itself is unacceptable as a standard because of the uncertainty involved in determining how much water is in fact delivered by equipment used to furnish sample water for reference purposes. The amount of moisture furnished must be previously known in order to ascertain whether the hydrogen detector or determinator is accurately calibrated. Typically, the small amount of moisture expected in a nuclear fuel pellet, which is less than one milligram of water, suggests a standard providing similar amounts of water. Since this amount of moisture is so small, delivery of the water in an appropriately sized container to apparatus used for analyzing the water content, without losing any of the water by evaporation, presents great logistics and control problems. Nevertheless, as the water of crystallization is used as a standard or reference the substance containing the water of crystallization must meet the following requirements:

a) It must not contain or have on its surface, any hydrogen other than that contained in the water of crystallization;

b) The water of crystallization must be capable of being extracted only at high temperatures to make certain that the substance will be dried of all surface moisture before measuring the hydrogen in the water of crystallization;

c) The substance containing the water of crystallization desirably must be in a form which will permit efficient and quick measurement of hydrogen; and

d) It must be non-toxic, both at ambient and elevated temperatures at which the water of crystallization is drawn off from the substance.

An exploration of substances, compounds and materials capable of satisfying the above requirements showed that many materials contained water of crystallization which could be extracted at elevated temperatures without destroying the material. The most desired substance found was kaolinite, $Al_2O_3 \cdot (SiO_2) \cdot 2H_2O$, which is a solid ceramic material having a small ratio of water to compound weight so that a relatively small amount of water could be delivered to the test apparatus without weighing out an unreasonably small quantity of the compound. The kaolinite contains 13.745% ± .008% water by weight when dried of surface moisture. All the hydrogen in kaolinite is contained in the water of crystallization and as a solid, it will have no evaporation losses. The water of crystallization can be dissociated and expelled or extracted from the kaolinite at a high temperature, i.e., above 900°C. This temperature is below the operating temperature of the LECO apparatus used for determining the amount of hydrogen in the water, but still sufficiently high to allow a sample of kaolinite to be dried of surface moisture without driving off the water of crystallization. Kaolinite is non-toxic, thus permitting its unrestricted use without the need to implement safeguard procedures to protect operating personnel. When raised to a temperature above 1000°C, the water of crystallization is driven off and the kaolinite changes its crystalline structure to become another non-toxic ceramic material, i.e., mullite.

To make certain that the LECO apparatus could accurately measure total water content of a standard with no significant difference from the theoretical, assuming an error risk of 5%, tests were conducted on two sets of ten kaolinite samples of various weights. These samples were dried in small amounts enclosed in pre-weighed tin capsules and were run on different days. The difference in gross and tare weights of a tin capsule gave the weight of the kaolinite inside and multiplying this weight by 13.745% gave the weight of water that would be extracted. The results in the Figure, "LECO Measurement of Fraction $H_2O$ of Kaolinite", shows that two samples of the second set were eliminated because of experimental error. A correction for the moisture background of the tin capsules was calculated and used in analysis of the data.

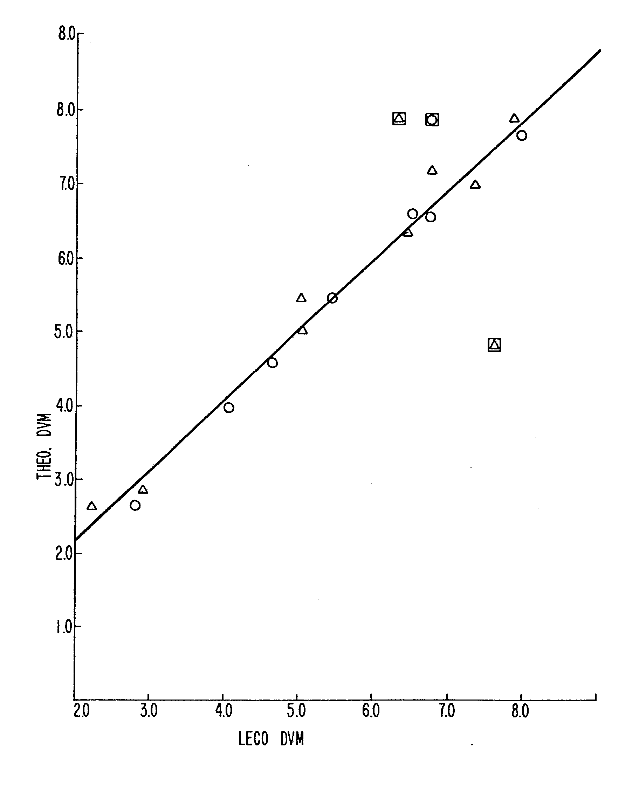

According to usual procedures, the eighteen LECO measurements were treated as eighteen determinations of a standard, namely the percent $H_2O$ in kaolinite, and were compared statistically to the theoretical value, 13.745% $H_2O$. The data shown in Figure 1 is graphed as "DVM Theoretical" versus "DVM Measured" in order to shown the linearity of the data and to disclose any outliers or samples beyond the test range. The results of the eighteen kaolinite standards shown on the graph include DVM units which represent the output of the LECO and which can be converted into PPM $H_2O$ when multiplied by 63 and divided by sample weight. The ordinate represents the DVM units expected from the water in each kaolinite standard. The abscissa represents the actual DVM reading for each standard. Each LECO DVM was also converted to percent $H_2O$ and the average of the eighteen samples was found not to be significantly different, at a 95% confidence level, from the theoretical. In view of both the chemical and physical characteristics of kaolinite, taken together with the ease of making and using samples thereof, it is apparent that it represents an excellent standard for PPM water and for determining the accurate calibration of the hydrogen measuring apparatus.

Having thus determined that the LECO hydrogen determinator accurately and precisely measure hydrogen alone and/or the hydrogen content in moisture it also can serve as a means for showing that the LECO apparatus is properly calibrated. As indicated above, the percentage water of crystallization in kaolinite is known and by using a relatively small amount of kaolinite, e.g., about .0007 grams, the water of crystallization can be extracted and the hydrogen content in the moisture accurately determined.

To determine the hydrogen content in a nuclear fuel pellet, having dimensions of about .366 inch diameter × .600 inch length, the pellet is dried of moisture and placed in the LECO apparatus and exposed to heat at about 2000°C. At these temperatures, the pellet characteristics are such that it fragments as a result of internal pressures and stresses and all of the moisture contained in the pellet is driven off and thus made available to the LECO hydrogen sensors. The hydrogen content in the moisture is thereupon measured and a digital readout on the apparatus visually displays the hydrogen content in the pellet.

## Claims

1. A method of determining the hydrogen content of nuclear fuel pellets wherein a nuclear fuel pellet whose hydrogen content is to be measured is placed into a hydrogen measuring apparatus in which said pellet is exposed to a temperature sufficient to assure the extraction of moisture from said pellet, and the content of hydrogen in the moisture extracted from said pellet is measured and a readout thereof is provided on said apparatus, characterized in that, before the water content of said fuel pellet is measured, kaolinite having a water of crystallization of a known amount is surface-dried and sealed and is then heated in said apparatus to the temperature at which the water of crystallization is driven from the kaolinite, the quantity of hydrogen in the moisture driven from the kaolinite is measured and compared with the quantity of hydrogen known to be in the water of crystallization of said kaolinite and the apparatus is then precisely adjusted so as to provide an accurate reading for the following measuring of the water content of the fuel pellet.

2. A method according to claim 1, characterized in that the fuel pellet is exposed to a temperature of about 2000°C to assure the release of all water from the pellet.

3. A method according to claim 1 or 2, characterized in that a small portion of the kaolinite is enclosed in a capsule having a melting temperature below the temperature at which the water of crystallization is withdrawn from the kaolinite so as to assure retention of the kaolinite in a dry environment until the capsule is melted during heating thereby to expose the kaolinite to the apparatus heat.

## Revendications

1. Procédé de dosage de la teneur en hydrogène de pastilles de combustible nucléaire dans laquelle une pastille de combustible nucléaire dont la teneur en hydrogène doit être mesurée est placée dans un appareil à mesurer l'hydrogène dans lequel ladite pastille est exposée à une température suffisante pour assurer l'extraction de l'humidité de ladite pastille, et la teneur en hydrogène de l'humidité extraite de ladite pastille est mesurée et une lecture de cette teneur est fournie par ledit appareil, caractérisé par le fait que, avant que la teneur en eau de ladite pastille de combustible soit mesurée, de la kaolinite ayant une eau de cristallisation en quantité connue est séchée en surface et scellée et est ensuite chauffée dans ledit appareil à la température à laquelle l'eau de cristallisation est chassée de la kaolinite, la quantité d'hydrogène, dans l'humidité chassée de la kaolinite est mesurée et comparée à la quantité d'hydrogène connue comme devant être dans l'eau de cristallisation de ladite kaolinite et l'appareil est ensuite calibré avec précision de façon à fournir une lecture précise de la mesure suivante de la teneur en eau de la pastille de combustible.

2. Procédé selon la revendication 1, caractérisé par le fait que la pastille de combustible est exposée à une température d'environ 2000°C pour assurer la libération de toute l'eau de la pastille.

3. Procédé selon les revendications 1 ou 2, caractérisé par le fait qu'une petite portion de la kaolinite est enfermée dans une capsule ayant une température de fusion inférieure à la température à laquelle l'eau de cristallisation est extraite de la kaolinite de façon à assurer la rétention de la kaolinite dans un environnement sec jusqu'à ce que la capsule soit fondue pendant le chauffage pour exposer de ce fait la kaolinite à la chaleur de l'appareil.

## Patentansprüche

1. Verfahren zum Bestimmen des Wasserstoffgehaltes eines Kernbrennstoffzylinders, bei dem ein Kernbrennstoffzylinder, dessen Wasserstoffgehalt gemessen werden soll, in ein Wasserstoffmessgerät eingebracht wird, in dem der Zylinder einer Temperatur von solcher Höhe ausgesetzt wird, dass mit Sicherheit alle Feuchtigkeit aus dem Zylinder freigesetzt wird, worauf der Wasserstoffgehalt in der freigesetzten Feuchtigkeit gemessen und von dem Messgerät angezeigt wird, dadurch gekennzeichnet, dass vor der Messung des Feuchtigkeits-gehaltes des Kernbrennstoffzylinders eine bestimmte Menge Kaolin, dass eine bekannte Menge Wasser im Kristallgitter enthält, äusserlich völlig getrocknet in das Messgerät eingeschlossen wird und dort auf eine Temperatur aufgeheizt wird, bei der das Kristallgitterwasser vom Kaolin ausgetrieben wird, dass die mit dem Wasser ausgetriebene Wasserstoffmenge gemessen und mit dem bekannten Sollwert für die Wassermenge im Kristallgitter des Kaolins verglichen wird und dass das Messgerät dann für eine gute Wiedergabe der folgenden Feuchtigkeits-gehaltmessung für einen Kernbrennstoffzylinder genau eingestellt wird.

2. Verfahren nach Anspruch 1, dadurch gekennzeichnet, dass der Kernbrennstoffzylinder einer Temperatur von etwa 200 Grad C ausgesetzt wird, um die vollkommene Austreibung des im Brennstoff enthaltenen Wassers sicherzustellen.

3. Verfahren nach Anspruch 1 oder 2, dadurch gekennzeichnet, dass eine kleine Menge Kaolin in eine Kapsel eingeschlossen wird, die aus einem Material besteht, dessen Schmelztemperatur unter der Temperatur liegt bei der das Kristallgitter-wasser aus dem Kaolin ausgetrieben wird, um damit sicherzustellen, dass das Kaolin trocken eingeschlossen bleibt, bis die Kapsel beim Heizvorgang schmilzt und erst dann das Kaolin der Gerätetemperatur voll ausgesetzt wird.